Europäisches Patentamt

European Patent Office

Office européen des brevets

(19)

(11) **EP 1 294 850 B1**

(12) **EUROPEAN PATENT SPECIFICATION**

(45) Date of publication and mention
of the grant of the patent:
**21.07.2004 Bulletin 2004/30**

(21) Application number: **01922126.6**

(22) Date of filing: **13.04.2001**

(51) Int Cl.⁷: **C12N 1/20**, A01N 63/04

(86) International application number:
**PCT/KR2001/000616**

(87) International publication number:
**WO 2001/083706 (08.11.2001 Gazette 2001/45)**

(54) **A MICROBIAL PESTICIDE ACTIVE AGAINST PLANT FUNGAL PATHOGENS AND PROCESS FOR PREPARATION THEREOF**

GEGEN PFLANZLICHE PILZPATHOGENE AKTIVES, MIKROBIELLES PESTIZID UND VERFAHREN ZUR HERSTELLUNG DESSELBEN

PESTICIDE MICROBIEN DIRIGE CONTRE LES PATHOGENES FONGIQUES DES VEGETAUX ET PROCEDE DE PREPARATION CORRESPONDANT

(84) Designated Contracting States:
**AT BE CH CY DE DK ES FI FR GB GR IE IT LI LU MC NL PT SE TR**

(30) Priority: **02.05.2000 KR 2000023498**
**16.03.2001 KR 2001013542**

(43) Date of publication of application:
**26.03.2003 Bulletin 2003/13**

(73) Proprietor: **Chung, Young-Ryun**
**Kyungsangnam-do, Jinju-city 660-321 (KR)**

(72) Inventors:
• **CHUNG, Young-Ryun**
**Kyungsangnam-do, Jinju-city 660-321 (KR)**
• **LEE, Son-Kug**
**Kyungsangnam-do, Guchang-gun 670-910 (KR)**

• **KIM, Ki-Woong**
**Pusan-city 618-300 (KR)**

(74) Representative: **Portal, Gérard et al**
**Cabinet Beau de Loménie**
**158, rue de l'Université**
**75340 Paris Cédex 07 (FR)**

(56) References cited:
EP-A- 0 466 133          DE-A- 3 600 394
JP-A- 4 029 905          JP-A- 9 087 122
US-A- 4 713 342          US-A- 4 748 021
US-A- 4 996 157

• **DAVET P ET AL: "The Use of Trochoderma in the Control of Soil Fungi" CROPU, XP002167234**

**EP 1 294 850 B1**

**Description**

**TECHNICAL FIELD**

The present invention relates, in general, to a novel *Trichoderma* species highly antagonistic to plant fungal pathogens, isolated from soil and, more particularly, to a biological fungicide highly effective against plant fungal pathogens, which is not harmful to natural ecosystems and free of mammalian toxicity. Also, the present invention is concerned with a method for preparing the biological fungicide.

**BACKGROUND ART**

Together with noxious insects and weeds, plant diseases are the main factors that threaten the stable production of crops. Plants are taken ill by various pathogens. Representative of them are fungi. Most of the pathogens which form sclerotia, such as *Botrytis cinerea*, and *Rhizoctonia solani,* reside in the soil, causing a variety of diseases in a broad spectrum of crops. The fungal pathogens pass the winter as survival structures of sclerotia. In the next year, sclerotia act as primary infection sources. They survive from year to year in the soil or plant tissues even under severe conditions. Because sclerotia are more resistant to the activity of antagonistic microorganisms than are spores and mycelia, the survival structures are highly difficult to eradicate. *Cladosporium fulvum*, which is a kind of leaf mold, and *Alternaria solani*, which causes early blight, exert their pathogenic activity in various vegetables. At present, various germicides are available, but their misuse and abuse bring about an increase in the drug resistance of pathogens, resulting in a significant decrease in germ control. Accordingly, there remains a need for a sterilizing method that is safe and effective.

Biological control methods taking advantage of microbial agents are environmentally safe and effective. Since the early 1970s, active and extensive research has been directed to biological control methods all over the world. Recently, ten or more microbial germicides have been made commercially available. Further, since environmentally safe sustainable agriculture is now recognized as preferable, microbial agents have been intensively studied. Representative examples of the microbial agents that have been commercially available indude BINAB T, prepared from mixtures of *Trichoderma harziaum* and *T. polysporum,* for use in the control of internal decay of wood utility poles (1988, E.R. Butts International); GlioGard, derived from *Gliocladium virens*, for use in the control of seedling disease of ornamental and bedding plants (1990, W. R. Grace Co.); F-stop (1991, Kodak) and Trichodex (1997, developed in Israel), both derived from *T. harziaum;* KODIAK, a seed treatment derived from *Bacillus subtilis* (1990, Gustafson); DAGGER, derived from *Pseudomanas fluorescens,* for use in the control of damping-off caused by *Rhizoctonia* and *Pythium* (1990, Ecogen Inc.); BLUE CIRCLE, a seed treatment agent for various crops, derived from *P. cepacia* (1990, Stine Microbial Products); and MYCOSTOP, useful for controlling Fusarium-caused wilt disease and damping-off, derived from the antagonistic fungus *Streptomyces griseviridis* (1985, Kemira Oy Bicontrol of Finland). Of them, microbial agents using *Trichoderma harzianum* were found to be excellent in control effect and product safety and, in many countries, much effort has been made to isolate locally adapted strains and develop them into microbial products.

*T. harzianum* kills pathogens by perforating and infiltrating their mycelia when in physical contact with them. In this regard, the biofungicidal microorganism secretes chitinase and β-1,3-glucanase. Purified enzymes from *T. harzianum* were found to be inhibitory of various plant pathogens, including *R. solani* and *B. cinerea.* In addition, *Trichoma viride*, *Gliocladium roseum*, and *Penicillium sp.* are reported to have fungicial activity against *B. cinerea* and *R. solani* as demonstrated in greenhouse and field tests. In order to increase the persistent activity of *T. harizianum* in various natural environments, a substrate that the microbe can characteristically utilize is needed, or there must be provided conditions necessary for the antagonistic microorganism to predominate in a substrate, lest other microorganisms or target pathogens predate it. Upon mass production and formulation of antagonistic microorganisms, another consideration for selecting culture media is economy.

Conventional microbial agents taking advantage of antagonistic microbes are widely used for the control of plant diseases caused by fungi, nematodes, and viruses, and for the protection of the plants from vermin and weeds. According to literature, the mechanisms of biological control that antagonistic microorganisms exert on their corresponding targets are elucidated by the antibiotic functions of the antibiotic materials that they secrete, the parasitic and phagocytic functions of cell wall decomposing enzymes, the decrease in the population or pathogenicity of the target organisms through the competition for nutrients and niches, and the cross protection and resistance induction in host cells by use of non-pathogens or attenuated pathogens. For example, *Trichoderma sp.* and *Gliocladium sp.* are used for the control of fungal diseases by taking advantage of their secretions, such as antibiotic materials, volatile materials, toxins, and special inhibitory materials. Recently, intensive and active attention has been paid to the antagonistic functions based on parasitic and phagocytic functions (see for example US-A-4 748 021 , US-A-4 713 342). Antagonistic microbes are known to secrete chitinase, glucanase and cellulase, all being cell wall decomposing enzymes which can act to cause the lysis of noxious pathogens.

## DISCLOSURE OF THE INVENTION

Leading to the present invention, the intensive and thorough research on the control of crop diseases without concomitant environmental destruction, conducted by the present inventors, resulted in the finding that a novel fungal species isolated from the soil is highly antagonistic to plant pathogens and its mycelia or spores can be formulated into environmentally friendly biological fungicides for use in the control of various plant diseases.

It is therefore an object of the present invention to provide a novel fungal species antagonistic to pathogenic fungi fatal to crops.

It is another object of the present invention to provide a biological fungicide for use in the control of crop diseases, which is highly potent and shows no environmental problems or toxicity to higher species.

It is a further object of the present invention to provide a method for preparing such a biological fungicide.

In accordance with an aspect of the present invention, there is provided a fungal species *Trichoderma harzianum* YC459 (accession No. KCTC 0772BP) antagonistic to plant pathogens.

In accordance with another aspect of the present invention, there is provided a biological fungicide for controlling plant pathogens, comprising the mycelia or spores of the fungus of claim 1, or a mixture thereof, as a fungicidally active ingredient.

In accordance with a further aspect of the present invention, there is provided a method of preparing a biological fungicide for controlling plant pathogens, in which mycelia or spores of the fungus species of claim 1 or a mixture thereof is formulated with an ordinary carrier.

## BEST MODES FOR CARRYING OUT THE INVENTION

In the present invention, a novel species antagonistic pathogenic fungi is isolated from the soil. Soil samples are taken from different regions, and dilutions are made in water. To grow fungal species, the dilutions were spread over potato-dextrose agar (PDA) plates containing streptomycin, followed by incubation at 28 °C. Five days are required for the formation of single colonies on the plates. The single colonies are aseptically transferred to PDA slant media and stored at 4 °C. The fungal colonies are assayed for antagonistic activity against pathogenic fungal species, including *B. cinerea, R. solani, C. fulvum* and *A. solani* by a substitution culture method. An observation is made of mycelium lysis at the contact interface between the pathogens and the putative antagonistic species under a microscope to select a fungal species which is the most inhibitory of the pathogens. This fungal species was identified as being a novel mutant of *Trichoderma harzianum*, named *T. harzianum* YC459, and deposited in the Korean Collection for Type Culture of Korea Research Institute of Bioscience and Biotechnology (KRIBB) with the accession number KCTC 0772BP on April 29, 2000.

It is necessary to quantitatively evaluate the inhibitory activity of the novel fungal species against pathogens. Spores are collected from cultures of the fugal species and suspended in water. Dilutions are made on the suspension to control the spore population. Then, the controlled spore suspension is applied to plants for assaying the control of the diseases caused by the pathogens.

In the case of the gray mold rot caused by *B. cinerea,* the spore suspension of the novel fungal species is sprayed over cucumber leaves. After 1 day, a spore suspension of the pathogenic fungus is inoculated into the leaves. The pots of the cucumber seedlings inoculated with the pathogen are placed in a moist chamber maintained at 20 °C for three days, followed by quantitative analysis of disease occurrence as a occurrence percentage of appearing spots.

The suppressive activity of the antagonistic microbe of the present invention against damping-off is assayed on radish. In this regard, a medium comprising potato slices and farm soil mixed with sterile water is prepared for culturing *R. solani,* a pathogen causative of damping-off. The medium in which the pathogen has been cultured is dried in the shade, powdered, and stored at 4 °C before use as an inoculum. A mixture of the pathogen inoculum and ordinary soil is placed, along with different amounts of the antagonistic microbe suspension, in plastic pots which are then sawed with four radish seeds. After one week of cultivation in a greenhouse, quantitative analysis is made of the disease occurrence in the radish.

Greenhouse and field tests of the fungicidal activity of the antagonistic microbe against various pathogenic fungi are conduced. For this, tomatoes are cultivated both in a greenhouse and a field. The tomatoes cultivated in a greenhouse and a field are treated with the spore suspension of the novel antagonistic microbe of the present invention and then exposed to pathogenic fungi, *B. cinerea* causing gray mold rot, *C. fulvum* causing leaf mold, and *A. solani* causing early blight, separately. The experiment is performed according to a randomized block design with three repeat measures. For comparison, procymidone for use in the control of gray mold rot, propineb for use in the control of leaf mold, and azoxystrobin for use in the control of early blight are employed. Morbidity is determined -by examining the infection of all tomatoes one by one or by a sampling method. A significance test is carried out according to Duncan's multiple range test (DMRT).

Also, the present invention pertains to the formulation of the antagonistic microbe of the present invention.

The antagonistic microbe *T. harzianum* YC459 is inoculated to PDA and cultured at 28 °C for 7 days to germinate spores which is then used to prepare spore suspensions for use as seed inoculums in mass culture. Wheat chaff, sawdust, rice bran and chaff are mixed in combinations, and yeast extract is added at an amount of 0.5 % by volume to each combination. The mixtures are added with water and autoclaved at 121 °C for 1 hour. Each of the sterilized media is aseptically mixed with 200 mL of the spore suspension, followed by culturing the microbe at 28 °C for 7 days. Biomasses of the antagonistic microbe are dried for three days at room temperature in the shade, powdered, and passed through a sieve with a hole size of 1 mm. The mycelium and spore powder is formulated with a bulking agent, such as peat moss, vermiculite, talc, zeolite, or kaolinite. For formulation, any of the excipients that are generally used for microbial agents may be selected.

A better understanding of the present invention may be obtained in light of the following examples which are set forth to illustrate, but are not to be construed to limit the present invention.

EXAMPLE 1: Identification of Antagonistic Microorganism and Assay for Antagonistic Activity

EXPERIMENTAL EXAMPLE 1-1: Isolation of Antagonistic Microorganism and Its Antagonistic Activity (in vitro Assay)

In order to isolate microorganisms antagonistic to *B. cinerea,* soils and barnyard manure were sampled in five different regions in Korea. 5 g of each of the samples was suspended in 45 mL of sterile water. After being agitated at 28 °C for 30 min, the suspension was diluted by a factor of up to $10^4$ in a soil dilution agar plate method. 0.1 mL-portions of the dilution were withdrawn and evenly spread over potato-dextrose agar (PDA) media (Difco) with 100 ppm of streptomycin, followed by incubation at 28 °C for 5 days. Single colonies of fungi grown on the agar plates were isolated and transferred to PDA slant media which were then stored at 4 °C. The isolated fungi were assayed for antagonistic activity against *B. cinerea*, *R. solani*, *C. fulvum* and *A. solani* by a substitution culture method in which the mycelium lysis at the contact face between the pathogens and the putative antagonistic species was observed under a microscope to select a fungal species which was the most inhibitory of the pathogens. This fungal species was identified to belong to *Trichoderma harzianum,* named *T. harzianum* YC459, and deposited in the Korean Collection for Type Culture of Korea Research Institute of Bioscience and Biotechnology (KRIBB) with the accession number KCTC 0772BP on April 29, 2000.

EXPERIMENTAL EXAMPLE 1-2: Assay of Antagonistic Microbe for Inhibitory Activity

To evaluate in vivo inhibitory activity of the antagonistic species *T. harzianum* YC459, isolated in Experimental Example 1, against pathogens, the novel microbe was applied to leaves 1 day before the inoculation of pathogens. Then, the antagonistic effect was monitored.

In this regard, the isolated antagonistic microbe was cultured on PDA medium at 28 °C for 10 days in an incubator, after which spores thus formed were collected by scraping with addition of 4-5 mL of sterile water. The spore suspensions thus obtained were controlled to have a spore density of $10^6$ or $10^7$/mL, on average, and sprayed over cucumber leaves in the two-leaf stage, in such a sufficient amount as to flow down. After the leaves were let to stand at room temperature for 1 day, a spore suspension with a spore density of $10^6$ or $10^7$/mL of *B. cinerea,* a pathogen causing gray mold rot, was inoculated into the leaves in such a sufficient amount as to flow down. The pots of the cucumber seedlings inoculated with the pathogen were placed for three days in a moist chamber maintained at 20 °C, followed by quantitatively measuring disease occurrence as an occurrence percentage of appearing spots. The pathogen used in this experiment was the strain JM42 that had been stored in the laboratory since its isolation from diseased tomato leaves in 1994. The cucumber seedlings planted in pots were cultured at 20 °C for 14 days in an incubator and spores formed on the surface of the incubator were collected in the same manner as above to prepare spore suspensions. The inhibitory activity of the antagonistic microbe against the disease caused by the pathogen was calculated according to the following equation.

$$\text{Control Value(\%)} = [1 - (\frac{\text{Attack rate of treated group}}{\text{Attack rate of untreated group}})] \times 100$$

All experiments were carried out with 4 pots in each of which 2 stalks of cucumber were planted. A significance test was performed according to Duncan's multiple range test (DMRT). The experiment results are given in Table 1, below.

TABLE 1

| Inhibitory Activity of *T. harzianum* YC459 Against the Occurrence of Gray Mold Rot on Cucumber | | |
|---|---|---|
| Treatment Conc. (Spores/mL Suspension) | Morbidity(%)* | Control Value(%) |
| $10^4$ | 32.9 C | 66.1 |
| $10^5$ | 40.0 C | 58.8 |
| $10^6$ | 19.1 B | 80.3 |
| $10^7$ | 9.9 A | 89.8 |
| 0 (untreated) | 97.0 D | - |

Note: *the same letters mean no significant differences at p=0.05 (DMRT).

Almost all members of the group not treated with the antagonistic microbe of the present invention died, showing a morbidity of 97 %. On the other hand, when applying the antagonistic microbe, the attack rate was decreased with increasing of the spore density of the suspension. The spore suspension of the antagonistic microbe exhibited the greatest inhibitory activity with a control value of 89.8 % at a spore density of $10^7$/mL.

EXAMPLE 2: Mass Culture and Formulation of the Antagonistic Microbe

The antagonistic microbe *T. harzianum* YC459, isolated in Example 1, was inoculated to PDA and cultured at 28 °C for 7 days to germinate spores which were then used to prepare spore suspensions for use as seed inoculums in mass culture, in the same manner as in Experimental Example 2. Wheat chaff, sawdust, rice bran and chaff were mixed in combinations of wheat chaff plus chaff, sawdust plus chaff, rice bran plus chaff, and rice bran plus sawdust, at the same amount, and yeast extract was added at an amount of 0.5 % by volume to each combination. The mixtures were individually added with water to a water content of 40 % and autoclaved at 121 °C for 1 hour in 5 L autoclavable vinyl packs, Erlenmeyer flasks, or sealable vessels. Each of the sterilized media was aseptically mixed with 200 mL of the spore suspension, and incubated at 28 °C for 7 days. The media were agitated once every other day during the incubation so as to induce uniform growth of mycelia and germinate spores in large quantities. Biomasses of the antagonistic microbe were dried for three days at room temperature in the shade, powdered, and passed through a sieve with a hole size of 1 mm. The mycelium and spore powder was formulated with a bulking agent, such as peat moss, vermiculite, talc, zeolite, or kaolinite. For formulation, any of the excipients that are generally used for microbial agents may be selected.

EXAMPLE 3: Suppressive Activity of the Antagonistic Microbe Against Occurrence of Damping-Off

Using radish, the antagonistic microbe was assayed for suppressive activity against damping-off. 50 g of potato slices and 450 g of farm soil were mixed along with 100 mL of sterile water in a 2L Elernmeyer flask, followed by autoclaving the medium at 121 °C for 1 hour. 20 fragments of the PDA on which *R. solani* was cultured for 5 days were inoculated to the medium, which was then incubated at 28 °C for 14 days. The medium was dried in the shade, powdered, and stored at 4 °C until use as an inoculum. 0.5 g of the pathogen inoculum was well mixed with 1L of ordinary soil, along with different amounts of the antagonistic microbe suspension prepared in the same manner as in Experimental Example 2. The mixtures were placed in four plastic pots with a dimension of 7 x 7 cm, each of which was sown with four radish seeds, followed by cultivation for one week in a greenhouse. After one week of the cultivation, severity of disease was discriminated according to four levels: 1 sound; 2 light brown lesion; 3 died of the disease after germination; and 4 died of the disease before germination, and morbidity was calculated as a percentage. Control values of the antagonistic microbe were calculated according to the equation given above. The results are given in Table 2, below.

TABLE 2

| Suppressive Effect of *T. harzianum* YC459 on Damping-Off | | |
|---|---|---|
| Spore suspension(mL/soil L) | Morbidity(%)* | Control value(%) |
| Untreated | 92.5 D | - |

Note: *the same letters mean no significant differences at p=0.05 (DMRT).

TABLE 2   (continued)

| Suppressive Effect of *T. harzianum* YC459 on Damping-Off | | |
|---|---|---|
| Spore suspension(mL/soil L) | Morbidity(%)* | Control value(%) |
| 5 | 75.6 D | 18.3 |
| 10 | 60.8 C | 34.3 |
| 50 | 30.5 B | 67.0 |
| 100 | 22.7 A | 75.5 |

Note: *the same letters mean no significant differences at p=0.05 (DMRT).

$$\text{Control Value(\%)} = [1-(\frac{\text{Attack rate of treated group}}{\text{Attack rate of untreated group}})] \times 100$$

EXAMPLE 4: Greenhouse and Field Tests of Fungicidal Activity of the Antagonistic Microbe

EXPERIMENTAL EXAMPLE 4-1: Inhibitory Activity of the Antagonistic Microbe Against Tomato Gray Mold Rot

To determine the fungicidal activity of the antagonistic microbe of the present invention against *B. cinerea* causing gray mold rot, tomato plants were cultivated in a greenhouse with a dimension of 23.7 m in length, 7.0 m in width and 2.3 m in height. The cultivation was performed according to the following schedule;
1999, Sep. 30 Seeding tomato
Oct. 10 1st Temporary planting
Oct. 22 2nd Temporary planting
Nov. 2 Planting in two rows at intervals of 20 cm
Dec. 22 Pollination
2000, Feb. 11 1st Treatment with the antagonistic microbe (spray amount 300 L/10a)
Feb. 20 2nd Treatment with the antagonistic microbe (spray amount 300 L/10a)
Mar. 2 3rd Treatment with the antagonistic microbe (spray amount 330 L/10a)
Mar. 22 Measuring mobidity

The tomato plants treated with the antagonistic microbe occupied an area of 5.4 m$^2$ and the experiment was performed in triplicate in a randomized block design. For comparison, Procymidone was sprayed as a control at a usual concentration. The spore suspension of the antagonistic microbe prepared in Example 2 was added directly to water to bring about 500- and 1,000-fold dilutions of the population. Morbidity was determined by examining the infection of all tomato plants one by one. A significance test was performed according to Duncan's multiple range test (DMRT).

Given in the following Table 3 are suppressive effects of the antagonistic microbe of the present invention on the occurrence of gray mold rot in tomatoes. As seen in Table 3, the untreated group showed a morbidity of 17.4 % on average. 500- and 1,000-fold dilutions of the antagonistic microbe suspension decreased the morbidity to 5.4 % and 6.6 %, respectively. Upon treatment with Procymidone, the morbidity remained at a level of 10.4 % on average. As for control value, it was measured to be 65.6 %, on average, for the antagonistic microbe-treated group, which was significantly higher than that of the untreated or Procymidone-treated group.

TABLE 3

| Suppressive Effect of *T. harzianum* YC459 on Gray Mold Rot in Tomato | | | | | |
|---|---|---|---|---|---|
| Dilution fold | Morbidity (%) | | | | Control value(%) |
| | 1 | 2 | 3 | Avg.* | |
| 1,000 | 4.5 | 8.3 | 7.1 | 6.6 A | 62.1 |
| 500 | 4.6 | 5.6 | 6.1 | 5.4 A | 69.0 |
| Procymidone | 12.3 | 10.6 | 8.2 | 10.4 B | 40.2 |
| Untreated | 18.5 | 19.2 | 14.4 | 17.4 C | - |

Note: * the same letters mean no significant differences at p=0.05 (DMRT).

$$\text{Control Value (\%)} = [1 - (\frac{\text{Attack rate of treated group}}{\text{Attack rate of untreated group}})] \times 100$$

EXPERIMENTAL EXAMPLE 4-2: Inhibitory Activity of the Antagonistic Microbe Against Leaf Mold

To determine the fungicidal activity of the antagonistic microbe of the present invention against *C. fulvum* causing leaf mold, tomato plants were cultivated in the same manner as in Experimental Example 4-1 in a greenhouse with a dimension of 23.7 m in length, 7.0 m in width and 2.3 m in height. The experiment was performed in triplicate in a randomized block design. For comparison, propineb was sprayed as a control at a usual concentration. Morbidity was determined by examining the infection of 20 stalks of tomatoes per repeat measure and attack frequency was calculated according to the following equation:

$$\text{Attack frequency(\%)} = \frac{\Sigma(\text{Infected Stock No. x Factor})}{4 \text{ x Total No. of stocks examined.}} \ x \ 100$$

wherein the factor is set to be: 0 when no leaves are infected; 1 when one forth of the total leaves are infected; 2 when half of the total leaves are infected and some of them are withered; 3 when three fourths of the total leaves are infected and half of them are withered; and 4 when two thirds are withered to death.

Given in the following Table 4 are suppressive effects of the antagonistic microbe of the present invention on the occurrence of leaf mold in tomatoes. As seen in Table 4, the untreated group showed a morbidity of 21.9 % on average. 500- and 1,000-fold dilutions of the antagonistic microbe suspension decreased the morbidity to 6.6 % and 6.8 %, respectively. Upon treatment with propineb, the morbidity remained at a level of as high as 21.9 % on average. As for control value, it was measured to range from 69 to 70 %, on average, for the antagonistic microbe-treated group, which was significantly higher than that of the untreated or propineb-treated group.

TABLE 4

| Suppressive Effect of *T. harzianum* YC459 on Leaf Mold in Tomato | | | | | |
|---|---|---|---|---|---|
| Dilution fold | Morbidity (%) | | | | Control value(%) |
| | 1 | 2 | 3 | Avg.* | |
| 1,000 | 6.5 | 6.9 | 7.1 | 6.8 A | 69.0 |
| 500 | 6.6 | 6.2 | 7.0 | 6.6 A | 69.9 |
| Propineb | 10.3 | 11.2 | 14.5 | 12.0 B | 42.9 |
| Untreated | 18.8 | 22.4 | 24.4 | 21.9 C | - |

Note: *the same letters mean no significant differences at p=0.05 (DMRT).

$$\text{Control Value (\%)} = [1 - (\frac{\text{Attack rate of treated group}}{\text{Attack rate of untreated group}})] \times 100$$

EXPERIMENTAL EXAMPLE 4-3: Inhibitory Activity of the Antagonistic Microbe Against Tomato Gray Mold Rot

To determine the fungicidal activity of the antagonistic microbe of the present invention against *A. solani* causing early blight, tomato was cultivated in a field. The cultivation was performed in the following schedule;
2000, May 4 Seeding potato
    Jun. 10 1st Treatment with the antagonistic microbe (spray amount 300 L/10a)
    Jul. 5 2nd Treatment with the antagonistic microbe (spray amount 300 L/10a)
    Jul. 13 Measuring mobidity
The experiment was performed in a randomized block design with three repeat measures. For comparison, azoxystrobin was sprayed as a control at a usual concentration. Morbidity was determined by examining the infection of 20 stocks of potatoes per repeat measure and attack frequency was calculated as in Experimental Example 4-2.
Given in the following Table 5 are suppressive effects of the antagonistic microbe of the present invention on the occurrence of early blight in potatoes. As seen in Table 5, the untreated group showed a morbidity of 21.7 % on average. 500- and 1,000-fold dilutions of the antagonistic microbe suspension reduced the morbidity to as low as 9.2 % and 9.5 %, respectively. Upon treatment with azoxystrobin, the morbidity was also reduced to 9.2 % on average. As for control value, it was measured to range 56.2 to 57.6 %, on average, for the antagonistic microbe-treated group, which was significantly higher than that of the untreated group.

TABLE 5

| Suppressive Effect of *T. harzianum* YC459 on Early Blight in Potato | | | | | |
|---|---|---|---|---|---|
| Dilution Fold | Morbidity (%) | | | | Control value(%) |
| | 1 | 2 | 3 | Avg.* | |
| 1,000 | 10.3 | 8.9 | 9.3 | 9.5 A | 56.2 |
| 500 | 9.8 | 8.1 | 9.6 | 9.2 A | 57.6 |
| Azoxytrobin | 9.3 | 10.2 | 8.2 | 9.2 A | 57.6 |
| Untreated | 22.8 | 22.9 | 19.4 | 21.7 C | - |

Note: *the same letters mean no significant differences at p=0.05 (DMRT).

$$\text{Control Value(\%)} = [1 - (\frac{\text{Attack rate of treated group}}{\text{Attack rate of untreated group}})] \times 100$$

## INDUSTRIAL APPLICABILITY

As described hereinbefore, the novel strain *Trichoderma harzianum* YC459 (accession No. KCTC 0772BP) isolated from soil is antagonistic to plant fungal pathogens *Botrytis cinerea*, *Thizoctonia solani*, *Cladosporium fulvum*, and *Alternaria solani*, showing excellent suppressive activity against gray mold rot, damping-off, leaf mold, and early blight. From the novel strain, there can be derived an environment-friendly biofungicide, which is not harmful to natural ecosystems and is free of mammalian toxicity. Therefore, the present invention is very useful for the agricultural chemicals and environmental industries.

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

## Claims

1.   A fungal species *Trichoderma harzianum* YC459 (accession No. KCTC 0772BP) antagonistic to plant pathogens.

2.   A biological fungicide for controlling plant pathogens, comprising the mycelia or spores of the fungus of claim 1, or a mixture thereof, as a fungicidally active ingredient.

3.   A method of preparing a biological fungicide for controlling plant pathogens, in which mycelia or spores of the fungus species of claim 1 or a mixture thereof is formulated with an ordinary carrier.

## Patentansprüche

1.   Pilz-Spezies Trichoderma Harzianum YC459 (Zugangsnummer KCTC 0772BP), die antagonistisch gegenüber Pflanzenpathogenen ist.

2.   Biologisches Fungizid zur Kontrolle von Pflanzenpathogenen, umfassend die Myzelien oder Sporen des Pilzes gemäß Anspruch 1 oder ein Gemisch davon als fungizidal aktives Ingredienz.

3.   Verfahren zur Herstellung eines biologischen Fungizids zur Kontrolle von Pflanzenpathogenen, bei welchem Myzelien oder Sporen der Pilz-Spezies gemäß Anspruch 1 oder ein Gemisch davon mit einem herkömmlichen Träger formuliert sind.

**Revendications**

1. Espèce fongique *Trichoderma harzianum* YC459 (n°. d'ordre KCTC 0772BP) antagoniste de pathogènes végétaux.

2. Fongicide biologique pour la lutte contre des pathogènes végétaux, comprenant les mycéliums ou spores du champignon de la revendication 1, ou un mélange de ceux-ci, comme ingrédient actif du point de vue fongicide.

3. Procédé de préparation d'un fongicide biologique pour la lutte contre des pathogènes végétaux où des micelles ou spores de l'espèce fongique de la revendication 1 ou un mélange de ceux-ci sont formulés avec un support ordinaire.